# EUROPEAN PATENT APPLICATION

(11) **EP 4 385 973 A1**
(43) Date of publication of application: **19.06.2024**
(21) Application number: 22213857.0
(22) Date of filing: 15.12.2022
(51) Int. Cl.: C07C 51/44, C07C 51/47, C07C 59/08

(54) **A METHOD AND A PLANT FOR PURIFYING LACTIC ACID**

(71) Applicant: Sulzer Management AG, 8401 Winterthur (CH)
(72) Inventor: VON SCALA, Claudia, 8606 Greifensee (CH); RIMA, Simonetta, 8008 Zürich (CH); FÄSSLER, Peter, 4123 Allschwil (CH); BACHMANN, Christian, 8548 Ellikon an der Thur (CH)
(74) Representative: Henkel & Partner mbB

(57) **Abstract**

The present invention relates to a method for purifying a crude aqueous lactic acid composition comprising the steps of:
i) heating and pressurizing the crude aqueous lactic acid composition so as to obtain a liquid, heated and pressurized crude aqueous lactic acid composition,
ii) flash evaporating the liquid, heated and pressurized crude aqueous lactic acid composition obtained in step i) so as to obtain crude lactic acid vapor,
iii) gradually condensing the crude lactic acid vapor obtained in step ii) by leading it through at least three successive condensers so as to obtain at least three condensed streams, one of which being a purified lactic acid stream.

## Description

The present invention relates to a method and a plant for purifying lactic acid.

Lactic acid is the monomer and thus an essential component of polylactic acid homopolymers and copolymers. Polymers of lactic acid are of particular interest, because they can be obtained from renewable resources and are mostly compostable and/or biodegradable. Moreover, the technological and physiochemical properties of these polymers come quite close to the properties of those polymers derived from fossil-based resources, which explains why these polymers are regarded as highly promising substitutes for the latter. Furthermore, polymers of lactic acid have a wide range of applications. For instance, polylactic acid is used in the biomedical field in chirurgical implants, in films, such as e.g. in packagings, in fibers, such as e.g. for garments, in hygienic articles, in carpets and in disposable plastic products, such as e.g. disposable cutlery or containers. In addition, polylactic acid has found wide application in composite materials, such as in fiber-reinforced plastics.

Generally, two alternative principal methods for synthesizing polylactic acid are known. The first principal method is the direct polycondensation of lactic acid to polylactic acid. The second principal method known for synthesizing polylactic acid is the ring-opening-polymerization of lactide, which is the cyclic diester of lactic acid. The lactide may be produced by condensation of two lactic acid molecules. Alternatively, the lactide may be produced by first prepolymerizing lactic acid and then subjecting the oligomer or prepolymer, respectively, to a depolymerization reaction. For any of the aforementioned polylactic acid production method, lactic acid is the starting material. Therefore, there is a demand for pure lactic acid. Lactic acid is predominantly prepared by fermentation of carbohydrates from biomass, such as starch, sugar or corn, resulting in a crude lactic acid composition, which is not pure enough to be used for synthesizing polylactic acid. Therefore, the so obtained crude lactic acid composition has to be purified before use.

A known method for purifying lactic acid is based on the precipitation of lactic acid in the form of metallic lactates followed by a neutralization reaction using a strong acid, such as sulfuric acid. However, this process is characterized by a comparable low yield and leads to lactic acid with a comparable poor quality. An alternative known method for purifying lactic acid is based on the esterification of lactic acid with an alcohol, the subsequent distillation of the ester and finally the hydrolysis of the distilled ester to lactic acid. Other known methods base on electrodialysis, extraction and/or ion exchange. However, all of these methods are comparable expensive. A comparable cheap alternative to these processes would be the distillation of lactic acid. However, the purification of lactic acid by classical distillation is not possible, because lactic acid, which is both an alcohol and an acid, interesteri-fies itself by autocatalytic reaction, once water is removed. Thereby, lactic acid oligomeric and thus nearly nonvolatile ester chains are formed, which do not fit for classical distillation. In order to nevertheless use distillation for the purification of lactic acid, US 6,489,508 B1 has proposed to first purify lactic acid by anionic and cationic ion exchange, before concentrating the lactic acid and distilling the concentrated, water-free lactic acid. However, this process is complex and connected with comparable high investment costs for the plant (CAPEX) and with comparable high operational costs (OPEX).

In view of this, the object underlying the present invention is to provide a process for purifying lactic acid, which requires comparable low investment costs for the plant and comparable low operational costs, and which nevertheless leads to pure lactic acid having, if at all, a low content of impurities, such as salts, proteins, sugars, yeast, glycerol etc.

In accordance with the present invention this object is satisfied by providing a method for purifying a crude aqueous lactic acid composition comprising the steps of:
i) heating and pressurizing the crude aqueous lactic acid composition so as to obtain a liquid, heated and pressurized crude aqueous lactic acid composition,
ii) flash evaporating the liquid, heated and pressurized crude aqueous lactic acid composition obtained in step i) so as to obtain crude lactic acid vapor,
iii) gradually condensing the crude lactic acid vapor obtained in step ii) by leading it through at least three successive condensers so as to obtain at least three condensed streams, one of which being a purified lactic acid stream.

This solution bases on the finding that by flash evaporating liquid, heated and pressurized crude aqueous lactic acid composition, the majority of the crude aqueous lactic acid composition evaporates instantaneously, i.e. within a very short time period, and thus sufficiently fast that a dimerization as well as oligomerization of the lactic acid is reliably avoided. This is due to the fact that the formation of oligomeric ester chains is relatively fast, but not an immediate reaction so that during the flash evaporation no or at least no measurable dimerization and oligomerization occurs. Furthermore, after the flash evaporation no or at least no measurable dimerization and oligomerization occurs, because the respective reaction rates in the gas phase are extremely slow. Before the flash evaporation, no or only few oligomerization occurs due to the water content, which is preferably 15% by weight or more, and the low temperature. After the condensation of the lactic acid, dimerization and oligomerization will occur to a certain extent. The so obtained purified lactic acid fraction may then, as subsequently described, be further purified, for instance by performing a stripping step, in which other light boiling acids, such as formic acid, acetic acid and water, are stripped off. Further laborious and costly purification steps, such as ionic exchange steps, extractions steps or electrodialysis steps, are thus not required. All in all, the method in accordance with the present invention requires comparable low investment costs for the plant and comparable low operational costs, but nevertheless leads to pure lactic acid having, if at all, a low content of impurities, such as salts, proteins, sugars, yeast, glycerol etc.

Liquid, heated and pressurized crude aqueous lactic acid composition means in accordance with the present invention any composition comprising lactic acid, wherein the composition is present in liquid form, has a temperature of above ambient temperature (i.e. 23°C) and has a pressure above atmospheric pressure (i.e. 101325 Pa).

Lactic acid oligomer means in accordance with the present invention a condensation product of lactic acid comprising two to 100 lactic acid units, whereas condensation products of lactic acid comprising more than 100 lactic acid units are considered in accordance with the present invention as polylactic acid. Thus, lactide, i.e. the cyclic dimer of lactic acid, is also considered as lactic acid oligomer.

Leading crude lactic acid vapor obtained in step ii) through at least three successive condensers means in accordance with the present invention that the crude lactic acid vapor is led through a first of the at least three successive condensers and is partially condensed therein. While the condensed portion is removed, the remaining vapor is led through a second of the at least three successive condensers and is partially condensed therein. While the condensed portion is removed, the remaining vapor is led through a third of the at least three successive condensers and is partially or fully condensed therein. While the condensed portion is removed, the possible remaining vapor is either removed or, if more than three successive condensers are present, led through a next of the more than three successive condensers.

The present invention is not particularly limited concerning the composition of the crude aqueous lactic acid composition, as far as it contains water and lactic acid. For instance, the crude aqueous lactic acid composition is produced by fermentation of carbohydrates from biomass, such as starch, sugar or corn. Preferably, the crude aqueous lactic acid composition, such as crude aqueous lactic acid composition obtained as fermentation broth, is filtered before subjecting it to step i) by using a (micro-)filter having an average pore size of 0.05 to 1.0 µm, more preferably of 0.1 to 0.5 µm and most preferably of 0.1 to 0.3 µm, in order to remove the microbiological cells. It if further preferred that as alternative to the aforementioned (micro-)filtration or after the aforementioned (micro-)filter, the crude aqueous lactic acid composition is filtered using a (nano-)filter having an average pore size of 1 to 100 nm and more preferably of 10 to 50 nm, in order to (also) remove at least most of the unreacted sugars, proteins and ions.

Moreover, it is preferred to adjust the water content of the micro- and/or nano-filtrated crude aqueous lactic acid composition, for instance by evaporating a part of the water, in order to reach a starting water concentration of 10 to 50% by weight, preferably of 15 to 30% by weight and more preferably of 15 to 20% by weight. For instance, the micro- and/or nano-filtrated and evaporated fermentation broth comprises, based on 100% by weigh of the crude aqueous lactic acid composition:
- 80 to 85% by weight of lactic acid and
- 15 to 20% by weight of water.

The crude aqueous lactic acid composition designates the composition, which is heated and pressurized so as to obtain a liquid, heated and pressurized crude aqueous lactic acid composition, which is then flash evaporated. This does not exclude that an aqueous lactic acid composition being produced by fermentation of carbohydrates from biomass is mixed with additional water and/or a recycle stream of heavies and/or other component(s), before it is heated and pressurized to the liquid, heated and pressurized crude aqueous lactic acid composition. Also in this case, the crude aqueous lactic acid composition designates the composition, which is heated and pressurized, i.e the composition being obtained after addition of the additional water and/or a recycle stream and/or other component(s).

In accordance with the present invention, the liquid, heated and pressurized crude aqueous lactic acid composition obtained in step i) is flash evaporated so as to obtain crude lactic acid vapor. Flash evaporation means in accordance with the present invention that the liquid crude composition is evaporated as a consequence of a pressure reduction of the heated and pressurized crude aqueous lactic acid composition. The pressure reduction may be obtained by any suitable means, as it is further described further below. The flash evaporation is thus, at least to a major degree, effected by the latent energy of the liquid, heated and pressurized crude aqueous lactic acid composition, i.e. the thermal energy and pressure energy of the liquid, heated and pressurized crude aqueous lactic acid composition. In light thereof, it is preferred that at least 70% of the energy required for the flash evaporation of the liquid, heated and pressurized crude aqueous lactic acid composition in step ii) is latent energy stored in the liquid, heated and pressurized crude aqueous lactic acid composition obtained in step i) as thermal energy and as pressure energy. Good results are in particular obtained, when at least 80%, more preferably at least 90%, still more preferably at least 95%, yet more preferably at least 99% and most preferably all of the energy required for the flash evaporation of the liquid, heated and pressurized crude aqueous lactic acid composition in step ii) is latent energy stored in the liquid, heated and pressurized crude aqueous lactic acid composition obtained in step i) as thermal energy and as pressure energy. This allows the liquid, heated and pressurized crude aqueous lactic acid composition to instantly evaporate, i.e. to evaporate within a very short time period, and thus much faster and much energy-efficient as in the case of leading non-heated, non-pressurized liquid over a heated surface. In other words, preferably at most 30%, more preferably at most 20%, still more preferably at most 10%, yet more preferably at most 5%, yet more preferably at most 1% and most preferably no energy at all is externally applied to the liquid, heated and pressurized crude aqueous lactic acid composition, such as in the form of a heated surface, just before and/or during the flash evaporation.

Good results are in particular obtained, when the crude aqueous lactic acid composition is heated in step i) so as to obtain a liquid, heated and pressurized crude aqueous lactic acid composition having a temperature of 140 to 220°C and preferably of 180 to 200°C, such as of about 190°C.

In a further development of the idea of the present invention, it is suggested that the crude aqueous lactic acid composition is pressurized in step i) so as to obtain a liquid, heated and pressurized crude aqueous lactic acid composition having a pressure of 0.2 to 5.0 MPa, preferably of 0.7 to 2.5 MPa and more preferably of 1.0 to 1.5, such as of about 1.2 MPa.

The heating and pressurization of the crude aqueous lactic acid composition may be performed in step i) in a single step or in two or more subsequent steps in any order. Thus, the heating may be performed before, after or during the pressurization of the crude aqueous lactic acid composition. While the heating of the crude aqueous lactic acid composition is preferably performed with one or more heat exchangers, the pressurization of the crude aqueous lactic acid composition may be performed with one or more pumps.

The flash evaporation in step ii) is preferably performed so that as much of the liquid, heated and pressurized crude aqueous lactic acid composition is evaporated as fast as possible. Good results are in particular obtained, when the flash evaporation of the liquid, heated and pressurized crude aqueous lactic acid composition is performed in step ii) so that at least 80% of the liquid, heated and pressurized crude aqueous lactic acid composition are evaporated within at most 1 second, more preferably within less than 0.75 second, even more preferably within at most 0.5 seconds and most preferably within 0.1 and 0.5 seconds.

The faster the liquid, heated and pressurized crude aqueous lactic acid composition is evaporated in step ii), the lower the chance for dimerization and oligomerization of the lactic acid.

It is proposed in a further development of the idea of the present invention that the flash evaporation in step ii) is performed in a vessel or column, the interior of which being adjusted to atmospheric or more preferably to sub-atmospheric pressure. Thereby, the pressure difference between the liquid, heated and pressurized crude aqueous lactic acid composition and the location, where it is flash evaporated, is maximized. On account of this reason it is particularly preferred that the pressure in the interior of the vessel or column is maintained or adjusted, respectively, to less than 100 kPa, more preferably to less than 10 kPa, even more preferably to less than 5 kPa and most preferably to less than 2 kPa, such as to about 1 kPa. There is in principle no lower limit for the pressure to be maintained or adjusted, respectively, in the interior of the vessel or column. However, for economical reasons the pressure in the interior of the vessel or column is preferably maintained or adjusted, respectively, to at least 0.1 kPa.

In order to obtain during step ii) an efficient flash evaporation, it is further preferred that the liquid, heated and pressurized crude aqueous lactic acid composition obtained in step i) is led in step ii) through a flash evaporation means, which comprises one or more openings or nozzles. The one or more openings or nozzles function as throttle and thus effect the pressure drop being necessary for effecting the flash evaporation. Good results are in particular obtained, when the pressure loss of the liquid, heated and pressurized crude aqueous lactic acid composition within the one or more openings or nozzles of the flash evaporation means is at least 0.1 MPa, more preferably at least 5.0 MPa, even more preferably at least 7.5 MPa and most preferably at least 10.0 MPa.

In accordance with a particular preferred embodiment of the present invention, the liquid, heated and pressurized crude aqueous lactic acid composition obtained in step i) is led in step ii) through a flash evaporation means in form of a ring-shaped pipe comprising an inlet for the liquid, heated and pressurized crude aqueous lactic acid composition as well as a plurality of openings or nozzles so as to flash evaporate the liquid, heated and pressurized crude aqueous lactic acid composition.

In accordance with an alternative, particular preferred embodiment of the present invention, the liquid, heated and pressurized crude aqueous lactic acid composition obtained in step i) is led in step ii) through a flash evaporation means in form of a T-shaped pipe comprising an inlet for the liquid, heated and pressurized crude aqueous lactic acid composition as well as two openings or nozzles so as to flash evaporate the liquid, heated and pressurized crude aqueous lactic acid composition. As set out further below, steps ii) and iii) are preferably performed in a vessel or a column, such as in a vertically arranged column having an essentially circular cross-section, wherein the vessel or column, respectively is margined by a wall. Preferably, the two openings or nozzles, respectively, are oriented so as to guide the liquid towards the vessel wall or column wall, respectively. It is possible to perform the method so that already a partially flashing of the liquid occurs inside the T-shaped pipe so that a mixture of liquid and vapor two openings or nozzles, respectively, towards the vessel wall or column wall, respectively. In order to protect the wall of the vessel or column, respectively, the ends of the T-shaped pipe comprising the openings or nozzles, respectively, are embodied so as to redirect the crude lactic acid vapor and possible remaining liquid after leaving the openings or nozzles, respectively, towards the circumference of the vessel or column, respectively. For this purpose, just downstream of each of the openings or nozzles, respectively, a V-shaped metal sheet may be arranged so as to redirect the outflowing liquid and vapor and to avoid that it hits the wall of the vessel or column, respectively, directly.

In accordance with still an alternative, particular preferred embodiment of the present invention, the liquid, heated and pressurized crude aqueous lactic acid composition obtained in step i) is led in step ii) through a flash evaporation means in form of a pipe comprising a plurality of arms, such as four to twenty and preferably eight to fifteen arms, each of the arms being provided with one or more openings or nozzles so as to flash evaporate the liquid, heated and pressurized crude aqueous lactic acid composition. As set out further below, steps ii) and iii) are preferably performed in a vessel or a column, such as in a vertically arranged column having an essentially circular cross-section, wherein the vessel or column, respectively is margined by a wall. Preferably, the flash evaporation means comprises one or more nozzles, wherein the one or more nozzles effect a pneumatic atomization or a full cone spray of the crude lactic acid vapor so as to flash evaporate the liquid, heated and pressurized crude aqueous lactic acid composition. Particularly preferred are full cone spray nozzles with a spray angle of 60 to 90°.

In accordance with yet another alternative, particular preferred embodiment of the present invention, the liquid, heated and pressurized crude aqueous lactic acid composition obtained in step i) is led in step ii) through a flash evaporation means in form of a cyclone. The cyclone may comprise a first cylindrical vessel with a first diameter and a second cylindrical vessel with a second diameter being bigger than the first diameter, with the second cylindrical vessel concentrically surrounding the first cylindrical vessel, wherein the first cylindrical vessel is arranged, seen in the vertical direction, displaced from the second cylindrical vessel. Preferably, the first cylindrical vessel is arranged so that, seen in the vertical direction, its lower end is arranged at a location corresponding to 30 to 70%, preferably 40 to 60%, such as about 50%, of the vertical length of the second cylindrical vessel, so that the lower half of the second cylindrical vessel does not contain the first cylindrical vessel, but only the upper half of the second cylindrical vessel. The upper end of the first cylindrical vessel is preferably arranged above the upper end of the second cylindrical vessel. The liquid, heated and pressurized crude aqueous lactic acid composition is introduced into the interior of the second cylindrical vessel so as to direct the liquid, heated and pressurized crude aqueous lactic acid composition towards the inner wall of the second cylindrical vessel so as to completely evaporate the crude aqueous lactic acid composition. The so obtained vapor of crude aqueous lactic acid composition is withdrawn from the flash evaporation means through the interior of the first cylindrical vessel.

In accordance with yet another alternative, particular preferred embodiment of the present invention, the liquid, heated and pressurized crude aqueous lactic acid composition obtained in step i) is led in step ii) through a flash evaporation means in form of a Schoepentoeter vessel. Such a vessel has in its plan view the shape of a trapeze, with the width of the vessel reducing in the direction from the inlet to the outlet. Both sides of the vessel are margined by deflector plates, which allow vapor to pass it.

In accordance with yet another alternative, particular preferred embodiment of the present invention, the liquid, heated and pressurized crude aqueous lactic acid composition obtained in step i) is led in step ii) through a flash evaporation means in form a vapor horn, which is a cylindrical vessel comprising an outer wall and concentrically therein an inner wall defining therebetween an annular channel, through which the liquid, heated and pressurized crude aqueous lactic acid composition obtained in step i) is led. The inner wall is formed of deflector plates, which allow vapor to pass it.

In accordance with the present invention, the crude lactic acid vapor obtained in step ii) is gradually condensed during step iii) by leading it through at least three successive condensers so as to obtain at least three condensed streams, one of which being a purified lactic acid stream. For this purpose, in step iii) in each of at least three successive condensers except the last condenser a portion of the vapor is condensed so as to obtain a condensed portion and a remaining vapor portion, wherein the condensed portion obtained in each of the at least three condensers is withdrawn as liquid and the remaining vapor portion of each of the at least three condensers (except for that of the last of the at least three condensers) is led to the next of the at least three condensers. In the last condenser, also a portion of the vapor may be condensed so as to obtain a condensed portion and a remaining vapor portion, or all of the vapor may be condensed so as to obtain only a condensed stream. If in the last condenser only a portion of the vapor is condensed, then the remaining vapor portion obtained after the last condenser is withdrawn.

Good results are in particular achieved, when the steps ii) and iii) are performed in a vessel or a column, such as in a vertically arranged column having an essentially circular cross-section, in which the at least three subsequent condensers are arranged vertically above each other. Thereby, both steps can be conducted in a comparable small and compact device.

Preferably, the column further comprises the flash evaporation means for flash evaporating the liquid, heated and pressurized crude aqueous lactic acid composition, wherein all of the at least three subsequent condensers are arranged above the flash evaporation means. Thereby, it is achieved in an efficient manner that the crude lactic acid vapor is gradually condensed in step iii) by leading it through at least three successive condensers so as to obtain three condensed streams.

In accordance with the present invention, the crude lactic acid vapor obtained in step ii) is gradually condensed in step iii) by leading it through at least three successive condensers so as to obtain at least three condensed streams, one of which being a purified lactic acid stream. In practice, it is sufficient that the crude lactic acid vapor is gradually condensed in step iii) by leading it through (exactly) three successive condensers. The lowest condenser condenses the heavies, i.e. the components of the crude lactic acid vapor having higher boiling points than lactic acid, whereas the second condenser, i.e. the condenser being arranged downstream of the first condenser or, if the condensers are all arranged vertically above each other, the condenser being arranged above the first condenser, condenses lactic acid in admixture with water and, if at all, only minor amounts of impurities, and the third and, if present, any further condenser condenses the water, small amounts of lactic acid and lights, i.e. the components of the crude lactic acid vapor having the lower boiling points than lactic acid. Residual lights and water remaining as vapor after the last condenser, if any, are removed as overhead stream.

Preferably, from the first of the at least three or of the exactly three successive condensers a condensed stream is withdrawn, which comprises lactic acid oligomers, lactide, proteins, metals and one or more organic dicarboxylic acids.

Moreover, it is preferred that from the second of the at least three or of the exactly three successive condensers a purified lactic acid condensed stream is withdrawn, which comprises lactic acid in admixture with water and, if at all, only minor amounts of impurities, such as lower carboxylic acids, such as formic acid and acetic acid.

In order to obtain a particular pure lactic acid product composition, it is suggested in a further development of the idea of the present invention that the purified lactic acid condensed stream being obtained in the second of the condensers is further purified so as to separate water and other possible impurities therefrom. Good results are for example achieved, when the purified lactic acid condensed stream being obtained in the second of the condensers is lead through a stripper column comprising, preferably at its bottom, a heat exchanger (or reboiler, respectively) for partially evaporate the purified lactic acid condensed stream so as to strip water and light organic molecules, preferably formic acid and acetic acid, from the purified lactic acid condensed stream. Instead of the heat exchanger or reboiler, respectively, or in addition to the heat exchanger or reboiler, respectively, steam may be injected into the stripper column so as to partially evaporate the purified lactic acid condensed stream so as to achieve the stripping effect. Alternatively to stripping, the purified lactic acid condensed stream may be subjected to a further distillation column comprising one or more stages.

Furthermore, it is preferred that from the third of the at least three or of the exactly three successive condensers a condensed stream is withdrawn, which comprises water, small amounts of lactic acid and lights, i.e. the components of the crude lactic acid vapor having the lower boiling points than lactic acid. The content of lactic acid in this condensed stream is typically less than 5% by weight and even not more than about 2% by weight, so that this stream contains small amounts of lactic acid, but is not at all a purified lactic acid composition.

Residual lights and water remaining as vapor after the last condenser are removed as overhead stream.

According to another aspect, the present invention relates to a plant for purifying a crude aqueous lactic acid composition comprising:
- one or more means for heating and pressurizing a crude aqueous lactic acid composition to a liquid, heated and pressurized crude aqueous lactic acid composition,
- a flash evaporation means for flash evaporating liquid, heated and pressurized crude aqueous lactic acid composition obtained in the means for heating and pressurizing a crude aqueous lactic acid composition,
- at least three successive condensers for gradually condensing the crude lactic acid vapor obtained in the flash evaporation means.

The plant may comprise one means functioning as means for heating as well as as means for pressurizing the crude aqueous lactic acid composition to a liquid, heated and pressurized crude aqueous lactic acid composition. Alternatively, the plant may comprise one means functioning as means for heating the crude aqueous lactic acid composition and one means functioning as means for pressurizing the crude aqueous lactic acid composition, whereas both means may be arranged in any order so as to produce a liquid, heated and pressurized crude aqueous lactic acid composition. While the means for heating the crude aqueous lactic acid composition preferably comprises one or more heat exchangers, the means for pressurizing the crude aqueous lactic acid composition preferably comprises one or more pumps.

In accordance with a particular preferred embodiment of the present invention, the plant comprises a column, in which the flash evaporation means and the at least three successive condensers are arranged. Preferably, the column is a vertically arranged column having an essentially circular cross-section, in which the at least three subsequent condensers are arranged vertically above each other. Thereby, both steps can be conducted in a comparable small and compact device.

As set out above, the flash evaporation means preferably comprises one or more openings or nozzles. Preferably, the flash evaporation means comprises one or more nozzles, wherein the one or more nozzles preferably effect a pneumatic atomization or a full cone spray of the crude lactic acid vapor so as to flash evaporate the liquid, heated and pressurized crude aqueous lactic acid composition.

In a further development of the idea of the present invention, it is proposed that the flash evaporation means is a ring-shaped pipe comprising a plurality of openings or nozzles.

In accordance with an alternative, particular preferred embodiment of the present invention, the flash evaporation means has the form of a T-shaped pipe comprising an inlet for the liquid, heated and pressurized crude aqueous lactic acid composition as well as two (outlet) openings or nozzles. More specifically, the flash evaporation means preferably comprises an inlet pipe section for the liquid, heated and pressurized crude aqueous lactic acid composition as well as two outlet pipe sections each comprising one or more openings or nozzles so as to flash evaporate the liquid, heated and pressurized crude aqueous lactic acid composition. Preferably, the flash evaporation means is arranged in a vessel or a column, such as in a vertically arranged column having an essentially circular cross-section, wherein the vessel or column, respectively is margined by a wall. Preferably, the two openings or nozzles, respectively, are oriented so as to guide the liquid or a mixture of liquid and vapor towards the vessel wall or column wall, respectively. In order to protect the wall of the vessel or column, respectively, it is further preferred that the ends of the T-shaped pipe comprising the openings or nozzles, respectively, are embodied so as to redirect the crude lactic acid vapor and possible remaining liquid after leaving the openings or nozzles, respectively, towards the circumference of the vessel or column, respectively. For this purpose, preferably just downstream of each of the openings or nozzles, respectively, a V-shaped metal sheet is arranged so as to redirect the outflowing liquid and vapor and to avoid that it hits the wall of the vessel or column, respectively, directly.

In accordance with still an alternative, particular preferred embodiment of the present invention, the flash evaporation means has the form of a pipe comprising a plurality of arms, such as four to twenty and preferably eight to fifteen arms, each of the arms being provided with one or more openings or nozzles so as to flash evaporate the liquid, heated and pressurized crude aqueous lactic acid composition. More specifically, the flash evaporation means preferably comprises an inlet pipe section for the liquid, heated and pressurized crude aqueous lactic acid composition, a distribution arm as well as a plurality of outlet pipe sections each comprising one or more openings or nozzles so as to flash evaporate the liquid, heated and pressurized crude aqueous lactic acid composition. The function of the distribution arm is to homogeneously distribute the distribution arm to the plurality of outlet arms. The distribution arm may be omitted so that the flash evaporation means only comprises an inlet pipe section for the liquid, heated and pressurized crude aqueous lactic acid composition and a plurality of outlet pipe sections each comprising one or more openings or nozzles. Moreover, it is preferred that the evaporation means is arranged in a vessel or a column, such as in a vertically arranged column having an essentially circular cross-section, wherein the vessel or column, respectively is margined by a wall.

In accordance with yet another alternative, particular preferred embodiment of the present invention, the flash evaporation means has the form of a cyclone. The cyclone may comprise a first cylindrical vessel with a first diameter and a second cylindrical vessel with a second diameter being bigger than the first diameter, with the second cylindrical vessel concentrically surrounding the first cylindrical vessel, wherein the first cylindrical vessel is arranged, seen in the vertical direction, displaced from the second cylindrical vessel. Preferably, the first cylindrical vessel is arranged so that, seen in the vertical direction, its lower end is arranged at a location corresponding to 30 to 70%, preferably 40 to 60%, such as about 50%, of the vertical length of the second cylindrical vessel, so that the lower half of the second cylindrical vessel does not contain the first cylindrical vessel, but only the upper half of the second cylindrical vessel. The upper end of the first cylindrical vessel is preferably arranged above the upper end of the second cylindrical vessel. During its operation, the liquid, heated and pressurized crude aqueous lactic acid composition is introduced into the interior of the second cylindrical vessel so as to direct the liquid, heated and pressurized crude aqueous lactic acid composition towards the inner wall of the second cylindrical vessel so as to completely evaporate the crude aqueous lactic acid composition. The so obtained vapor of crude aqueous lactic acid composition is withdrawn from the flash evaporation means through the interior of the first cylindrical vessel.

In accordance with yet another alternative, particular preferred embodiment of the present invention, the flash evaporation means has the form of a Schoepentoeter vessel. Such a vessel has in its plan view the shape of a trapeze, with the width of the vessel reducing in the direction from the inlet to the outlet. Both sides of the vessel are margined by deflector plates, which allow vapor to pass it.

In accordance with yet another alternative, particular preferred embodiment of the present invention, the flash evaporation means has the form a vapor horn, which is a cylindrical vessel comprising an outer wall and concentrically therein an inner wall defining therebetween an annular channel, through which the liquid, heated and pressurized crude aqueous lactic acid composition obtained in step i) is led. The inner wall is formed of deflector plates, which allow vapor to pass it.

Preferably, the at least three subsequent condensers are arranged in the column vertically above the flash evaporation means.

Subsequently, the present invention is described by means of illustrative, but not limiting figures, in which:
- Fig. 1: shows a schematic view of the plant for purifying lactic acid in accordance with one embodiment of the present invention.
- Fig. 2: shows a schematic view of a flash evaporation means being suitable to be used in a plant for purifying lactic acid in accordance with one embodiment of the present invention.
- Fig. 3: shows a schematic view of a flash evaporation means being suitable to be used in a plant for purifying lactic acid in accordance with one embodiment of the present invention.
- Fig. 4: shows a schematic view of a flash evaporation means being suitable to be used in a plant for purifying lactic acid in accordance with another embodiment of the present invention.
- Fig. 5: shows a schematic view of a flash evaporation means being suitable to be used in a plant for purifying lactic acid in accordance with another embodiment of the present invention.

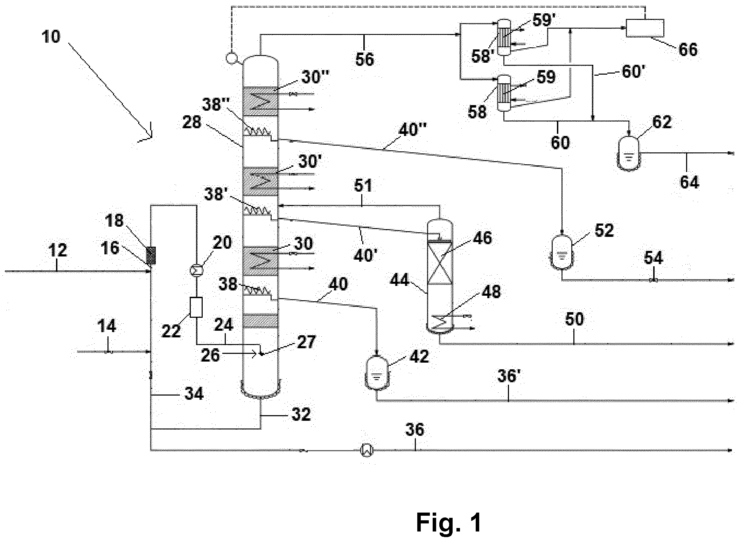

The plant 10 for purifying a crude aqueous lactic acid composition shown in figure 1 comprises an inlet 12 for lactic acid composition obtained by fermentation and an inlet 14 for water, which both are connected with each other to form the line 16 for crude lactic acid composition. The line 16 for crude lactic acid composition comprises a static mixer 18 for mixing the components, a heat exchanger 20 for heating the crude lactic acid composition and a pump 22 for pressurizing the crude lactic acid composition so as to form a liquid, heated and pressurized crude aqueous lactic acid composition. A line 24 for liquid, heated and pressurized crude lactic acid composition leads from the pump 22 to an evaporation means 26 comprising several nozzles, from which in figure 1 only one nozzle 27 is shown. The evaporation means 26 or nozzles 27, respectively, are arranged in the lower part of a column 28 and the column 28 further comprises three condensers 30, 30', 30", which are arranged vertically above each other and all above the evaporation means 26 or nozzles 27, respectively. In the bottom of the column 28, a bottom line 32 is arranged, which splits into a recycle line 34 leading into the line 16 for crude lactic acid composition and into a removal line 36 for heavies. Below each of the condensers 30, 30', 30", a collector 38, 38', 38" is arranged, from which a removal line 40, 40', 40" leads out of the column 28. The removal line 40 from the collector 38 of the lowermost condenser 30 leads into a vessel 42, from which in turn a removal line 36' for heavies leads out of the plant 10. The removal line 40' from the collector 38' of the second condenser 30' leads into a stripper column 44, which comprises a structured packing 46 and in its lower part a heat exchanger or reboiler 48, respectively. From the stripper column 44 a removal line 50 for purified lactic acid leads out of the plant 10, whereas a return line 51 for the stripped gas leads back into the column 28. The removal line 40" from the collector 38" of the uppermost condenser 30" leads into a vessel 52, from which in turn a removal line 54 for lights leads out of the plant 10. Finally, from the overhead of column 28 an overhead removal line 56 leads to two vessels 58, 58', each of which being provided with a freeze condenser 59, 59' and from each of which a removal line 60, 60' leads to a vessel 62, from which a removal line for lights leads out of the plant 10. Finally, the plant 10 comprises a vacuum unit 66, which controls the vacuum in the column 28, i.e. which adjusts and maintains the vacuum in the column 28 at a desired value.

During the operation of the plant 10, lactic acid composition obtained by fermentation is fed via line 12 and water is fed via line 14 into the plant 10. The lactic acid composition, the water and heavies including dimers and oligomers of lactic acid removed from the column 28 via line 32 are led via line 16 into the static mixer 18, in which a homogeneous mixture is formed from the components. The so formed crude lactic acid composition is heated in the heat exchanger 20 and pressurized in the pump 22 so as to form a liquid, heated and pressurized crude aqueous lactic acid composition, which is led via line 24 into the flash evaporation means 26 or nozzles 27, respectively, of the column 28 and is flash evaporated immediately after leaving the nozzles 27. Remaining liquid heavies are withdrawn from the bottom of the column 28 via line 32, from which a major part is withdrawn from the plant 10 via line 36, whereas a smaller part is recycled via line 34 into line 16. The flash evaporated crude lactic acid vapor is successively led to the condensers 30, 30', 30". In each of the condensers 30, 30', 30", a portion of the crude lactic acid vapor is condensed, which is collected in the respective collector 38, 38', 38" and removed from the column 28 via lines 40, 40', 40". The condensed liquid withdrawn via line 40, which comprises mainly heavies, such as oligomerized lactic acid, metals, proteins, succinic acid, oxalic acid and the like, is led into vessel 42, from which it is in turn removed via line 36'. The condensed liquid withdrawn via line 40' from the collector 38' of the second condenser 30' is led into the stripper column 44, in which water and lights, such as formic acid and acetic acid, are removed from the lactic acid and led via return line 51 back into the column 28. The purified lactic acid is removed via line 50. The condensed liquid withdrawn via line 40" from the collector 38" of the uppermost condenser 30" is led into the vessel 52 and removed therefrom via line 54 from the plant 10. Finally, the overhead fraction is led via line 56 into the vessels 58, 58', wherein the condensed portions generated by the freeze condensers 59, 59' are led via lines 60, 60' into vessel 62 and from there via line 64 out of the plant 10.

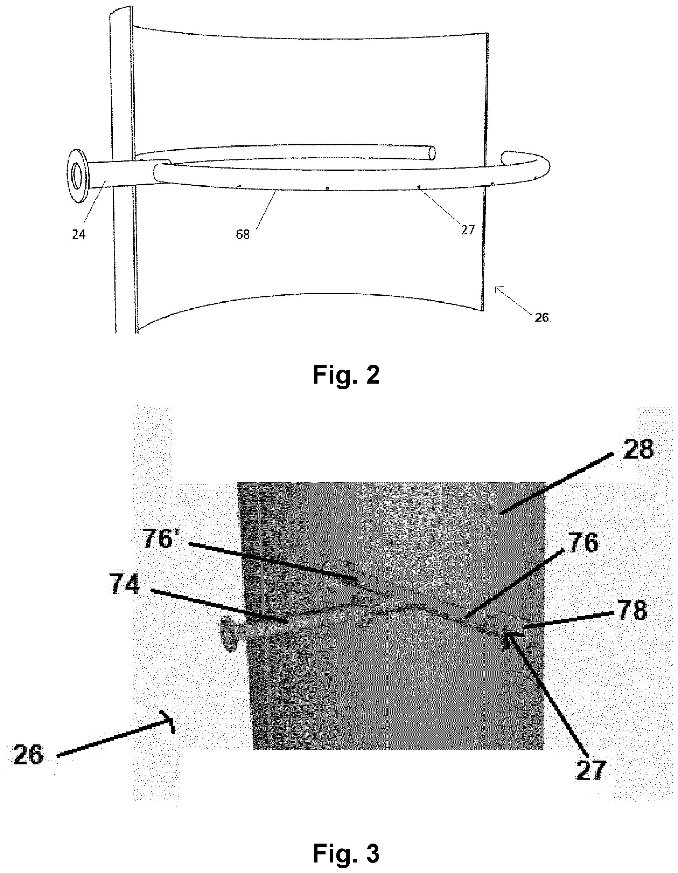

Figure 2 shows a schematic view of a flash evaporation means 26 being suitable to be used in a plant 10 for purifying lactic acid in accordance with the present invention. The flash evaporation means 26 is a ring-shaped pipe 68 comprising an inlet 24 for the liquid, heated and pressurized crude aqueous lactic acid composition as well as a plurality of openings or nozzles 27 so as to flash evaporate the liquid, heated and pressurized crude aqueous lactic acid composition.

The flash evaporation means 26 shown in figure 3 has the form of a T-shaped pipe and is arranged in a column 28. The T-shaped pipe comprises an inlet pipe section 74 for the liquid, heated and pressurized crude aqueous lactic acid composition as well as two outlet pipe sections 76, 76' each comprising an opening or nozzle 27 so as to flash evaporate the liquid, heated and pressurized crude aqueous lactic acid composition. Downstream of each of the openings or nozzles 27, respectively, a V-shaped metal sheet 78 is arranged.

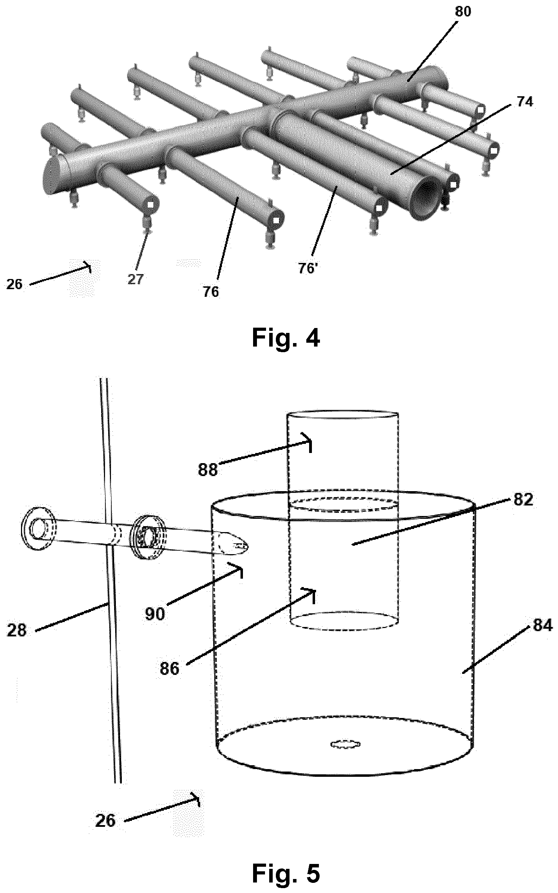

The flash evaporation means 26 shown in figure 4 has the form of a pipe comprising an inlet pipe section 74 for the liquid, heated and pressurized crude aqueous lactic acid composition, a distribution arm 80 as well as twelve outlet pipe sections 76, 76' each comprising one or more openings or nozzles 27 so as to flash evaporate the liquid, heated and pressurized crude aqueous lactic acid composition. The function of the distribution arm is to homogeneously distribute the distribution arm to the plurality of outlet arms.

The flash evaporation means 26 shown in figure 5 is a cyclone. The cyclone 26 comprises a first cylindrical vessel 82 having a first diameter and a second cylindrical vessel 84 having a second diameter being bigger than the first diameter. The second cylindrical vessel 84 concentrically surrounds the first cylindrical vessel 82, wherein the first cylindrical vessel 82 is arranged, seen in the vertical direction, displaced from the second cylindrical vessel 84. More specifically, the first cylindrical vessel 82 is arranged so that, seen in the vertical direction, its lower end 86 is arranged at about 50% of the vertical length of the second cylindrical vessel 84, so that the lower half of the second cylindrical vessel 84 does not contain the first cylindrical vessel 82. The upper end 88 of the first cylindrical vessel 82 is arranged above the upper end 90 of the second cylindrical vessel 84. During its operation, the liquid, heated and pressurized crude aqueous lactic acid composition is introduced into the interior of the second cylindrical vessel 84 so as to direct the liquid, heated and pressurized crude aqueous lactic acid composition towards the inner wall of the second cylindrical vessel 84 so as to completely evaporate the crude aqueous lactic acid composition. The so obtained vapor of crude aqueous lactic acid composition is withdrawn from the flash evaporation means 26 through the upper end 88 of the first cylindrical vessel 82.

### Reference numerals

- 10: Plant
- 12: Inlet for lactic acid composition obtained by fermentation
- 14: Inlet for water
- 16: Line for crude lactic acid composition
- 18: Static mixer
- 20: Heat exchanger
- 22: Pump
- 24: Line for liquid, heated and pressurized crude lactic acid composition
- 26: Evaporation means
- 27: Opening(s)/Nozzle(s)
- 28: Column
- 30,30',30": Condenser
- 32: Bottom line
- 34: Recycle line
- 36,36': Removal line for heavies
- 38,38',38": Collector
- 40,40',40": Removal line
- 42: Vessel
- 44: Stripper column
- 46: Structured packing
- 48: Heat exchanger / reboiler
- 50: Removal line for purified lactic acid
- 51: return line for the stripped gas
- 52: Vessel
- 54: Removal line for lights
- 56: Overhead removal line

- 58,58': Vessel
- 59, 59': Freeze condenser
- 60,60': Removal line
- 62: Vessel
- 64: Removal line
- 66: Vacuum unit
- 68: Ring-shaped pipe
- 74: Inlet pipe section
- 76, 76': Outlet pipe section
- 78: V-shaped metal sheet
- 80: Distribution arm
- 82: First cylindrical vessel
- 84: Second cylindrical vessel
- 86: Lower end of the first cylindrical vessel
- 88: Upper end of the first cylindrical vessel
- 90: Upper end of the second cylindrical vessel

## Claims

1. A method for purifying a crude aqueous lactic acid composition comprising the steps of:
i) heating and pressurizing the crude aqueous lactic acid composition so as to obtain a liquid, heated and pressurized crude aqueous lactic acid composition,
ii) flash evaporating the liquid, heated and pressurized crude aqueous lactic acid composition obtained in step i) so as to obtain crude lactic acid vapor,
iii) gradually condensing the crude lactic acid vapor obtained in step ii) by leading it through at least three successive condensers so as to obtain at least three condensed streams, one of which being a purified lactic acid stream.

2. The method in accordance with claim 1, wherein the crude aqueous lactic acid composition has been prepared by filtering a crude composition using a filter having an average pore size of 0.05 to 1.0 µm, more preferably of 0.1 to 0.5 µm and most preferably of 0.1 to 0.3 µm and/or a filter having an average pore size of 1 to 100 nm and more preferably of 10 to 50 nm, and preferably by then adjusting the water content of the micro- and/or nano-filtrated crude aqueous lactic acid composition to 10 to 50% by weight, preferably to 15 to 30% by weight and more preferably to 15 to 20% by weight.

3. The method in accordance with claim 1 or 2, wherein at least 70%, preferably at least 80%, more preferably at least 90%, still more preferably at least 95%, yet more preferably at least 99% and most preferably all of the energy required for the flash evaporation of the liquid, heated and pressurized crude aqueous lactic acid composition in step ii) is latent energy stored in the liquid, heated and pressurized crude aqueous lactic acid composition obtained in step i).

4. The method in accordance with any of the preceding claims, wherein the crude aqueous lactic acid composition is heated in step i) so as to obtain a liquid, heated and pressurized crude aqueous lactic acid composition having a temperature of 140 to 220°C and preferably of 180 to 200°C.

5. The method in accordance with any of the preceding claims, wherein the crude aqueous lactic acid composition is pressurized in step i) so as to obtain a liquid, heated and pressurized crude aqueous lactic acid composition having a pressure of 0.2 to 5.0 MPa, preferably of 0.7 to 2.5 MPa and more preferably of 1.0 to 1.5.

6. The method in accordance with any of the preceding claims, wherein the flash evaporation of the liquid, heated and pressurized crude aqueous lactic acid composition is performed in step ii) so that at least 80% of the liquid, heated and pressurized crude aqueous lactic acid composition are evaporated within at most 1 second.

7. The method in accordance with any of the preceding claims, wherein in step ii) the liquid, heated and pressurized crude aqueous lactic acid composition obtained in step i) is led through a flash evaporation means comprising one or more openings or nozzles, wherein the pressure loss of the liquid, heated and pressurized crude aqueous lactic acid composition within the one or more openings or nozzles is at least 0.1 MPa, preferably at least 5.0 MPa, more preferably at least 7.5 MPa and most preferably at least 10.0 MPa so as to flash evaporate the liquid, heated and pressurized crude aqueous lactic acid composition.

8. The method in accordance with any of the preceding claims, wherein in step ii) the liquid, heated and pressurized crude aqueous lactic acid composition obtained in step i) is led through a flash evaporation means being a cyclone or a ring-shaped pipe comprising a plurality of openings or nozzles so as to flash evaporate the liquid, heated and pressurized crude aqueous lactic acid composition, and/or wherein in step ii) the liquid, heated and pressurized crude aqueous lactic acid composition obtained in step i) is led through a flash evaporation means comprising one or more nozzles, wherein the one or more nozzles effect a pneumatic atomization or a full cone spray of the crude lactic acid vapor so as to flash evaporate the liquid, heated and pressurized crude aqueous lactic acid composition.

9. The method in accordance with any of the preceding claims, wherein in step iii) in each of at least three successive condensers a part of the vapor is condensed so as to obtain a condensed portion and a remaining vapor portion, wherein the condensed portion obtained in each of the at least three condensers is withdrawn as liquid and the remaining vapor portion of each of the at least three condensers except for that of the last of the at least three condensers is led to the next of the at least three condensers.

10. The method in accordance with any of the preceding claims, wherein the flash evaporation of the liquid, heated and pressurized crude aqueous lactic acid composition in step ii) as well as the gradual condensations of the crude lactic acid vapor in step iii) are performed in a column, in which the at least three subsequent condensers are arranged vertically above each other, wherein the column further comprises a flash evaporation means for flash evaporating the liquid, heated and pressurized crude aqueous lactic acid composition, wherein all of the at least three subsequent condensers are arranged above the flash evaporation means.

11. The method in accordance with any of the preceding claims, wherein in step iii) the crude lactic acid vapor is gradually condensed by leading it through three successive condensers so as to obtain three condensed streams, wherein from the first of the three successive condensers a condensed stream is withdrawn, which comprises lactic acid oligomers, lactide, proteins, metals and one or more organic dicarboxylic acids.

12. The method in accordance with claim 11, wherein from the second of the three successive condensers a purified lactic acid condensed stream is withdrawn, wherein the purified lactic acid condensed stream is preferably further purified by leading it through a stripper column comprising a heat exchanger for partially evaporate the purified lactic acid condensed stream so as to strip water and light organic molecules, preferably formic acid and acetic acid, from the purified lactic acid condensed stream.

13. A plant for purifying a crude aqueous lactic acid composition comprising:
- one or more means for heating and pressurizing a crude aqueous lactic acid composition to a liquid, heated and pressurized crude aqueous lactic acid composition,
- a flash evaporation means for flash evaporating liquid, heated and pressurized crude aqueous lactic acid composition obtained in the means for heating and pressurizing a crude aqueous lactic acid composition,
- at least three successive condensers for gradually condensing the crude lactic acid vapor obtained in the flash evaporation means.

14. The plant in accordance with claim 13, wherein the plant comprises a column, in which the flash evaporation means and the at least three successive condensers are arranged.

15. The plant in accordance with claim 13 or 14, wherein the flash evaporation means comprises one or more openings or nozzles, wherein preferably the flash evaporation means is a ring-shaped pipe comprising a plurality of openings or nozzles, wherein preferably below the ring-shaped pipe a ring-shaped collector is provided, wherein the ring-shaped collector preferably has an u-shaped groove.
